# EUROPEAN PATENT SPECIFICATION

(11) **EP 1 390 710 B1**
(45) Date of publication and mention of the grant of the patent: **08.03.2006**
(21) Application number: 02731359.2
(22) Date of filing: 11.04.2002
(51) Int. Cl.: C07H 21/04, C12Q 1/44, C12Q 1/68, C12P 19/34

(54) **AMPLIFICATION AND DETECTION OF MYCOPLASMA PNEUMONIAE**
AMPLIFIKATION UND NACHWEIS VON MYCOPLASMA PNEUMONIAE
AMPLIFICATION ET DETECTION DE MYCOPLASMA PNEUMONIAE

(30) Priority: 13.04.2001 US 283601 P
(43) Date of publication of application: 25.02.2004
(73) Proprietor: Becton, Dickinson and Company, Franklin Lakes, New Jersey 07417-1880 (US)
(72) Inventor: FINN, Stefanie, Owings Mills, MD 21117 (US); PRICE, James, A., Jr., Lutherville, MD 21093 (US); HELLYER, Tobin, J., Westminster, MD 21157 (US)
(74) Representative: Weber, Thomas
(86) International application number: PCT/US2002/011630
(87) International publication number: WO 2002/086441

(56) References cited:
- EP-A- 0 943 691
- US-A- 5 817 463
- US-B1- 6 277 582
- RULAND ET AL.: 'Sequence divergence in the ORF6 gene of mycoplasma pneumoniae' JOURNAL OF BACTERIOLOGY vol. 176, no. 17, September 1994, pages 5202 - 5209, XP002050945

### SEQUENCE LISTING

<110> BECTON, DICKINSON AND COMPANY
<120> AMPLIFICATION AND DETECTION OF MYCOPLASMA PNEUMONIAE
<130> P-5292.70
<140>
   <141>
<150> 60/283,601
   <151> 2001-04-13
<160> 31
<170> PatentIn Ver. 2.1
<210> 1
   <211> 41
   <212> DNA
   <213> Artificial Sequence
<220>
   <223> Description of Artificial Sequence: Primer
<400> 1
   cgattccgct ccagacttct cgggaatgcc ttgagttttg a 41
<210> 2
   <211> 38
   <212> DNA
   <213> Artificial Sequence
<220>
   <223> Description of Artificial Sequence: Primer
<400> 2
   cgattccgct ccagacttct cgggcaaccc cggaccca 38
<210> 3
   <211> 39
   <212> DNA
   <213> Artificial Sequence
<220>
   <223> Description of Artificial Sequence: Primer
<400> 3
   accgcatcga ttgactgtct cgggagaccc ggaagtgtc 39
<210> 4
   <211> 39
   <212> DNA
   <213> Artificial Sequence
<220>
   <223> Description of Artificial Sequence: Primer
<400> 4
   accgcatcga ttgactgtct cgggcaccgg tcaactttc 39
<210> 5
   <211> 16
   <212> DNA
   <213> Artificial Sequence
<220>
   <223> Description of Artificial Sequence: Primer
<400> 5
   ttcggaccaa agtaat 16
<210> 6
   <211> 14
   <212> DNA
   <213> Artificial Sequence
<220>
   <223> Description of Artificial Sequence: Primer
<400> 6
   aatggggttg ctca 14
<210> 7
   <211> 15
   <212> DNA
   <213> Artificial Sequence
<220>
   <223> *Description of Artificial Sequence: Primer*
<400> 7
   gacatagctg gcgaa 15
<210> 8
   <211> 14
   <212> DNA
   <213> Artificial Sequence
<220>
   <223> Description of Artificial Sequence: Primer
<400> 8
   atccaggggt tcat 14
<210> 9
   <211> 42
   <212> DNA
   <213> Artificial Sequence
<220>
   <223> Description of Artificial Sequence: Primer
<400> 9
   acgttagcca ccatacggat gaaggcactt aatggctcgc ag 42
<210> 10
   <211> 41
   <212> DNA
   <213> Artificial Sequence
<220>
   <223> Description of Artificial Sequence: Primer
<400> 10
   acgttagcca ccatacggat tcaccggctt taagctcgat a 41
   <210> 11
   <211> 35
   <212> DNA
   <213> Artificial Sequence
<220>
   <223> Description of Artificial Sequence: Probe
<400> 11
   tagtgcccga gcactacgtt agccaccata cggat 35
   <210> 12
   <211> 28
   <212> DNA
   <213> Artificial Sequence
<220>
   <223> Description of Artificial Sequence: Primer
<400> 12
   tacaggatcc acgagttcgg atcaaggt 28
   <210> 13
   <211> 28
   <212> DNA
   <213> Artificial Sequence
<220>
   <223> Description of Artificial Sequence: Primer
<400> 13
   acgtaagctt tcgaatccag gggttcat 28
   <210> 14
   <211> 213
   <212> DNA
   <213> Artificial Sequence
<220>
   <223> Description of Artificial Sequence: Consensus sequence
<220>
   <221> modified_base
   <222> (198)..(213)
   <223> a, t, c or g
<400> 14
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaagnnn nnnnnnnnnn nnn                              213
<210> 15
   <211> 202
   <212> DNA
   <213> Mycoplasma pneumoniae
<400> 15
   ggatccacga gttcggatca aagtaatacc aaccaaaatg ccttgagttt tgatacccaa 60
   gaatcacaga aggcacttaa tggctcgcag agtggatctt ctgacacttc cgggtctaac 120
   tcccaagact tcgccagcta tgtcctcatc tttaaagccg cgcccagggc cacgtgggtg 180
   tttgaacgca agattaagtt gg                                          202
<210> 16
   <211> 201
   <212> DNA
   <213> Mycoplasma pneumoniae
<400> 16
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaagttg g                                           201
<210> 17
   <211> 186
   <212> DNA
   <213> Mycoplasma pneumoniae
<400> 17
   gatccacgag ttcggatcaa agtaatacca cccaaaatgc cttgagtttt gacacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaac                                                            186
<210> 18
   <211> 182
   <212> DNA
   <213> Mycoplasma pneumoniae
<400> 18
   atccacgagt tcggatcaaa gtaataccac ccaaaatgcc ttgagttttg acacccaaga 60
   atcacagaag gcacttaatg gctcgcagag tggatcttct gacacttccg ggtctaactc 120
   ccaagacttc gccagctatg tcctcatctt taaagccgcg cccagggcca cgtgggtgtt 180
   tg                                                                182
<210> 19
   <211> 200
   <212> DNA
   <213> Mycoplasma pneumoniae
<400> 19
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaagttg                                             200
<210> 20
   <211> 213
   <212> DNA
   <213> Mycoplasma pneumoniae
<400> 20
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaagttg gcgttgccct acg                              213
<210> 21
   <211> 197
   <212> DNA
   <213> Mycoplasma pneumoniae
<400> 21
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaag                                                197
<210> 22
   <211> 197
   <212> DNA
   <213> Mycoplasma pneumoniae
<400> 22
   gatccacgag ttcggatcaa agtaatacca accaaaatgc cttgagtttt gatacccaag 60
   aatcacagaa ggcacttaat ggctcgcaga gtggatcttc tgacacttcc gggtctaact 120
   cccaagactt cgccagctat gtcctcatct ttaaagccgc gcccagggcc acgtgggtgt 180
   ttgaacgcaa gattaag                                                197
<210> 23
   <211> 248
   <222> DNA
   <213> Artificial Sequence
<220>
   <223> Description of Artificial Sequence: Consensus sequence
<220>
   <221> modified_base
   <222> (247)..(248)
   <223> a, t, c or g
<400> 23
   ctcgtcgaac aacccgtgac cccttacacc ccgaatgcgg ggttagcccg ggtgaatggg 60
   gttgctcagg atacggttca ttttggttcg ggtcaagaat cgagttggaa ttcccaacgt 120
   tcccaaaaag gccttaaaaa caaccccgga cccaaagccg tcaccggctt taagctcgat 180
   aagggccgcg cgtaccggaa gctgaatgaa agttgaccgg tgtatgaacc cctggattcg 240
   aaagctnn                                                          248
<210> 24
   <211> 247
   <212> DNA
   <213> Mycoplasma pneumoniae
<400> 24
   ctcgtcgaac aacccgtgac cccttacacc ccgaatgcgg ggttagcccg ggtgaatggg 60
   gttgctcagg atacggttca ttttggttcg ggtcaagaat cgagttggaa ttcccaacgt 120
   tcccaaaaag gccttaaaaa caaccccgga cccaaagccg tcaccggctt taagctcgat 180
   aagggccgcg cgtaccggaa gctgaatgaa agttgaccgg tgtatgaacc cctggattcg 240
   aaagctt                                                           247
<210> 25
   <211> 247
   <212> DNA
   <213> Mycoplasma pneumoniae
<400> 25
   ctcgtcgaac aacccgtgac cccttacacc ccgaatgcgg ggttagcccg ggtgaatggg 60
   gttgctcagg atacggttca ttttggttcg ggtcaagaat cgagttggaa ttcccaacgt 120
   tcccaaaaag gccttaaaaa caaccccgga cccaaagccg tcaccggctt taagctcgat 180
   aagggccgcg cgtaccggaa gctgaatgaa agttgaccgg tgtatgaacc cctggattcg 240
   aaagctt                                                           247
<210> 26
   <211> 216
   <212> DNA
   <213> Mycoplasma pneumoniae
<400> 26
   gaccccttac accccgaatg cggggttagc ccgggtgaat ggggttgctc aggatacggt 60
   tcattttggt tcggatcaag aatcgagttg gaattcccaa cgttcccaaa aaggccttaa 120
   aaacaacccc ggacccaaag ccgtcaccgg ctttaagctc gataagggcc gcgcgtaccg 180
   gaagctgaat gaaagttgac cggtgtatga acccct                          216
<210> 27
   <211> 228
   <212> DNA
   <213> Mycoplasma pneumoniae
<400> 27
   cccttacacc ccgaatgcgg ggttagcccg ggtgaatggg gttgctcagg atacggttca 60
   ttttggttcg gatcaagaat cgagttggaa ttcccaacgt tcccaaaaag gccttaaaaa 120
   caaccccgga cccaaagccg tcaccggctt taagctcgat aagggccgcg cgtaccggaa 180
   gctgaatgaa agttgaccgg tgtatgaacc cctggattcg aaagctta             228
<210> 28
   <211> 262
   <212> DNA
   <213> Mycoplasma pneumoniae
<400> 28
   aaccctccaa gacctcctcg tcgaacaacc cgtgacccct tacaccccga atgcggggtt 60
   agcccgggtg aatggggttg ctcaggatac ggttcatttt ggttcgggtc aagaatcgag 120
   ttggaattcc caacgttccc aaaaaggcct taaaaacaac cccggaccca aagccgtcac 180
   cggctttaag ctcgataagg gccgcgcgta ccggaagctg aatgaaagtt gaccggtgta 240
   tgaacccctg gattcgaaag ct                                         262
<210> 29
   <211> 255
   <212> DNA
   <213> Mycoplasma pneumoniae
<400> 29
   aagacctcct cgtcgaacaa cccgtgaccc cttacacccc gaatgcgggg ttagcccggg 60
   tgaatggggt tgctcaggat acggttcatt ttggttcggg tcaagaatcg agttggaatt 120
   cccaacgttc ccaaaaaggc cttaaaaaca accccggacc caaagccgtc accggcttta 180
   agctcgataa gggccgcgcg taccggaagc tgaatgaaag ttgaccggtg tatgaacccc 240
   tggattcgaa agctt                                                 255
<210> 30
   <211> 261
   <212> DNA
   <213> Mycoplasma pneumoniae
<400> 30
   aaccctccaa gacctcctcg tcgaacaacc cgtgacccct tacaccccga atgcggggtt 60
   agcccgggtg aatggggttg ctcaggatac ggttcatttt ggttcgggtc aagaatcgag 120
   ttggaattcc caacgttccc aaaaaggcct taaaaacaac cccggaccca aagccgtcac 180
   cggctttaag ctcgataagg gccgcgcgta ccggaagctg aatgaaagtt gaccggtgta 240
   tgaacccctg gattcgaaag c                                           261
<210> 31
   <211> 203
   <212> DNA
   <213> Mycoplasma pneumoniae
<400> 31
   tagcccgggt gaatggggtt gctcaggata cggttcattt tggttcgggt caagaatcga 60
   gttggaattc ccaacgttcc caaaaaggcc ttaaaaacaa ccccggaccc aaagccgtca 120
   ccggctttaa gctcgataag ggccgcgcgt accggaagct gaatgaaagt tgaccggtgt 180
   atgaacccct ggattcgaaa gct                                         203

## Description

### FIELD OF THE INVENTION

The present invention relates to methods for determining the presence or absence of *Mycoplasma pneumoniae* in respiratory samples or other patient specimens or.culture samples. The method involves using nucleic acid primers to amplify specifically a target sequence within the ORF6 gene, preferably using one of the techniques of Strand Displacement Amplification (SDA), thermophilic Strand Displacement Amplification (tSDA) or fluorescent real time tSDA.

### BACKGROUND OF THE INVENTION

*M. pneumoniae* is predominantly a pathogen of the human respiratory tract and can cause bronchitis, pharyngitis and atypical pneumonia. It most commonly infects older children and young adults. Standard laboratory methods for diagnosis of *M. pneumoniae* include culture and serology. Both methods have disadvantages; *M. pneumoniae* is fastidious and requires 1 to 3 weeks to culture, while serology is insensitive and non-specific. Nucleic acid amplification methods for the detection of *M. pneumoniae* potentially offer the advantages of speed and improved sensitivity and specificity.

Physical mapping, as described by Wenzel, et al. (1988, *Nucl. Acids Res.* 16: 8323-8336), and sequencing of the complete genome, as described by Himmelreich, et al. (1996, *Nucl. Acids Res.* 24: 4420-4449), of *M. pneumoniae* have been performed. Several mycoplasma membrane proteins are known to be involved in the attachment of *M*. *pneumoniae* to host cells. These include the P1, HMW1 and HMW3 proteins as well as the 40 and 90 kDa expression products of the ORF6 gene, all of which are associated together in a transmembrane protein complex (Sperker, et al., 1991, *Mol. Microbiol.* 5: 299-306; Layh-Schmitt, 1993, *Zentralbl Bakteriol* 278: 287-295; Layh-Schmitt, et al., 1999, *FEMS Microbiol. Lett.* 174: 143-149; Layh-Schmitt, et al., 2000, *Microbiol.* 146: 741-747). The ORF6 gene is one of two open reading frames that flank the P1 attachment protein gene. An operon-like organization has been proposed for this region of the *M. pneumoniae* genome with the order ORF4-ORF5/P1-ORF6 (Su, et al., 1987, *Infect. Immun.* 55: 3023-3029). The 3950bp ORF6 gene contains a repetitive element (RepMP5) of 1900bp that is repeated a total of eight times throughout the *M*. *pneumoniae* genome (Ruland, et al., 1994, *J. Bacteriol.* 176: 5202-5209). The importance of the ORF6 gene in the pathogenicity of the organism makes it a potentially useful target for development of *M*. *pneumoniae-specific* diagnostic tests. Selection of the RepMP5 element within the ORF6 gene as a target for such assays also has the advantage that sensitivity may be enhanced relative to that which can be achieved by targeting a single copy sequence within the *M. pneumoniae* genome.

Nucleic acid amplification is a powerful technology, which allows rapid detection of specific target sequences and it is therefore a promising technology for the rapid detection and identification of *M*. *pneumoniae.* The oligonucleotide primers of the present invention are applicable to nucleic acid amplification and detection of *M. pneumoniae.*
The following terms are defined herein as follows:

An amplification primer is a primer for amplification of a target sequence by extension of the primer after hybridization to the target sequence. Amplification primers are typically about 10-75 nucleotides in length, preferably about 15-50 nucleotides in length. The total length of an amplification primer for SDA is typically about 25-50 nucleotides. The 3' end of an SDA amplification primer (the target binding sequence) hybridizes at the 3' end of the target sequence. The target binding sequence is about 10-25 nucleotides in length and confers hybridization specificity on the amplification primer. The SDA amplification primer further comprises a recognition site for a restriction endonuclease 5' to the target binding sequence. The recognition site is for a restriction endonuclease which will nick one strand of a DNA duplex when the recognition site is hemimodified, as described by G. Walker, et al. (1992, *Proc. Natl. Acad. Sci. USA* 89:392-396 and 1992, *Nucl. Acids Res.* 20:1691-1696). The nucleotides 5' to the restriction endonuclease recognition site (the "tail") function as a polymerase repriming site when the remainder of the amplification primer is nicked and displaced during SDA. The repriming function of the tail nucleotides sustains the SDA reaction and allows synthesis of multiple amplicons from a single target molecule. The tail is typically about 10-25 nucleotides in length. Its length and sequence are generally not critical and can be routinely selected and modified. As the target binding sequence is the portion of a primer which determines its target-specificity, for amplification methods which do not require specialized sequences at the ends of the target the amplification primer generally consists essentially of only the target binding sequence. For example, amplification of a target sequence according to the invention using the Polymerase Chain Reaction (PCR) will employ amplification primers consisting of the target binding sequences of the amplification primers described herein. For amplification methods that require specialized sequences appended to the target other than the nickable restriction endonuclease recognition site and the tail of SDA (e.g., an RNA polymerase promoter for Self-Sustained Sequence Replication (3SR), Nucleic Acid Sequence-Based Amplification (NASBA), Transcription-Based Amplification System (TAS) or Rolling Circle Amplification (RCA)), the required specialized sequence may be linked to the target binding sequence using routine methods for preparation of oligonucleotides without altering the hybridization specificity of the primer.

A bumper primer or external primer is a primer used to displace primer extension products in isothermal amplification reactions. The bumper primer anneals to a target sequence upstream of the amplification primer such that extension of the bumper primer displaces the downstream amplification primer and its extension product.

The terms target or target sequence refer to nucleic acid sequences to be amplified. These include the original nucleic acid sequence to be amplified, the complementary second strand of the original nucleic acid sequence to be amplified and either strand of a copy of the original sequence which is produced by the amplification reaction. These copies serve as amplifiable targets by virtue of the fact that they contain copies of the sequence to which the amplification primers hybridize.

Copies of the target sequence that are generated during the amplification reaction are referred to as amplification products, amplimers or amplicons.

The term extension product refers to the copy of a target sequence produced by hybridization of a primer and extension of the primer by polymerase using the target sequence as a template.

The term species-specific refers to detection, amplification or oligonucleotide hybridization to a species of organism or a group of related species without substantial detection, amplification or oligonucleotide hybridization to other species of the same genus or species of a different genus.

The term assay probe refers to any oligonucleotide used to facilitate detection or identification of a nucleic acid. Detector probes, detector primers, capture probes, signal primers and reporter probes as described below are examples of assay probes.

A signal primer comprises a 3' target binding sequence that hybridizes to a complementary sequence in the target and further comprises a 5' tail sequence that is not complementary to the target (the adapter sequence). The adapter sequence is an indirectly detectable marker selected such that its complementary sequence will hybridize to the 3' end of the reporter probe described below. The signal primer hybridizes to the target sequence at least partially downstream of the hybridization site of an amplification primer. The signal primer is extended by the polymerase in a manner similar to extension of the amplification primers. Extension of the amplification primer displaces the extension product of the signal primer in a target amplification-dependent manner, producing a single-stranded product comprising a 5' adapter sequence, a downstream target binding sequence and a 3' binding sequence specific for hybridization to a flanking SDA amplification primer. Hybridization and extension of this flanking amplification primer and its subsequent nicking and extension creates amplification products containing the complement of the adapter sequence which may be detected as an indication of target amplification.

A reporter probe according to the present invention functions as a detector oligonucleotide and comprises a label which is preferably at least one donor/quencher dye pair, i.e., a fluorescent donor dye and a quencher for the donor fluorophore. The label is linked to a sequence or structure in the reporter probe (the reporter moiety) which does not hybridize directly to the target sequence. The sequence of the reporter probe 3' to the reporter moiety is selected to hybridize to the complement of the signal primer adapter sequence. In general, the 3' end of the reporter probe does not contain sequences with any significant complementarity to the target sequence. If the amplification products containing the complement of the adapter sequence described above are present, they can then hybridize to the 3' end of the reporter probe. Priming and extension from the 3' end of the adapter complement sequence allows the formation of the reporter moiety complement. This formation renders the reporter moiety double-stranded, thereby allowing the label of the reporter probe to be detected and indicating the presence of or the amplification of the target.

The term amplicon refers to the product of the amplification reaction generated through the extension of either or both of a pair of amplification primers. An amplicon may contain exponentially amplified nucleic acids if both primers utilized hybridize to a target sequence. Alternatively, amplicons may be generated by linear amplification if one of the primers utilized does not hybridize to the target sequence. Thus, this term is used generically herein and does not imply the presence of exponentially amplified nucleic acids.

### SUMMARY OF THE INVENTION

The present invention provides oligonucleotide primers that can be used for amplification of a target sequence found in *M. pneumoniae.* More specifically, the target sequence comprises a segment of the ORF6 gene. The amplification primers have been designed for high-efficiency, high-specificity amplification at elevated temperatures, such as in tSDA and the PCR, however, they are also useful.in lower-temperature amplification reactions such as conventional SDA, 3SR, TAS, NASBA or RCA. An oligonucleotide reporter probe that hybridizes to the complement of target specific signal primers is used to detect the amplification products.

The oligonucleotides of the invention may be used after culture as a means for confirming the identity of the cultured organism. Alternatively, they may be used for the detection and identification of *M. pneumoniae* in clinical samples from humans or animals using known amplification methods. In either case, the inventive oligonucleotides and assay methods provide a means for rapidly discriminating between *M*. *pneumoniae* and other microorganisms, allowing the practitioner to identify this microorganism rapidly without resorting to the more traditional procedures customarily relied upon. Such rapid identification of the specific etiological agent involved in an infection provides information that can be used to determine appropriate action within a short period of time.

### SUMMARY OF THE SEQUENCES

SEQ ID NOs: 1-2 are sequences of oligonucleotides used as upstream primers for amplification of a sequence within the ORF6 gene. SEQ ID NOs: 3-4 are sequences of oligonucleotides used as downstream primers for amplification of a sequence within the ORF6 gene. SEQ ID NOs: 5-6 are sequences of oligonucleotides used as upstream bumper primers for SDA amplification. SEQ ID NOs: 7-8 are sequences of oligonucleotides used as downstream bumper primers for SDA amplification. SEQ ID NOs: 9-10 are sequences of signal primers for amplification and detection of a sequence within the ORF6 gene. SEQ ID NO: 11 is a sequence for a reporter probe designed for detection of a sequence within the ORF6 gene when used in conjunction with any of the aforementioned signal primers. SEQ ID NO: 12 is a sequence of an oligonucleotide used as an upstream primer for PCR amplification of a sequence within the ORF6 gene. SEQ ID NO: 13 is a sequence of an oligonucleotide used as a downstream primer for PCR amplification of a sequence within the ORF6 gene.

### BRIEF DESCRIPTION OF THE DRAWINGS

The various objects, advantages and novel features of the present invention will be readily understood from the following detailed description when read in conjunction with the appended drawings in which:
Fig. 1 illustrates detection of a *M. pneumoniae* nucleic acid ORF6 gene target sequence in strand displacement amplification (SDA) reaction according to the method of the invention.
Fig. 2 illustrates the generation of a 529 bp amplification product when SEQ ID NOs: 12 and 13 are used in the PCR amplification of *M. pneumoniae* nucleic acid ORF6 gene target sequence.
Fig. 3 illustrates the lack of cross-reactivity of SEQ ID NOs: 12 and 13 when used in the PCR amplification of the nucleic acid of organisms phylogenetically related to *M. pneumoniae.*
Fig. 4 illustrates the alignment of the target binding sequences of SEQ ID NOs: 1, 3, 5, 7 and 9 in SDA amplification of the ORF6 gene from several *M. pneumoniae* strains.
Fig. 5 illustrates the alignment of the target binding sequences of SEQ ID NOs: 2, 4, 6, 8 and 10 in SDA amplification of the ORF6 gene from several *M. pneumoniae* strains.

### DETAILED DESCRIPTION OF THE INVENTION

The present invention relates to oligonucleotides, amplification primers and signal primers that exhibit specificity for *M. pneumoniae* in nucleic acid amplification reactions. Also provided are methods for detecting and identifying *M. pneumoniae* nucleic acids using the oligonucleotides of the invention. The preferred methods are to use SDA, tSDA or homogeneous real time fluorescent tSDA. These methods are known to those skilled in the art from references such as U.S. Patent No. 5,547,861, U.S. Patent No. 5,648,211, U.S. Patent No. 5,846,726, U.S. Patent No. 5,919,630, U.S. Patent No. 5,928,869, U.S. Patent No. 5,958,700, U.S. Patent No. 5,935,791, U.S. Patent No. 6,054,279, U.S. Patent No. 6,130,047, U.S. Patent Application Serial No. 09/590,061, filed June 08, 2000, and U.S. Patent Application Serial No. 09/602,996, filed June 23, 2000.

The primers of the present invention were designed based on an analysis of ORF6 gene sequence data from the M129 strain referenced in Genbank Accession # NC 000912. As shown in Table 1, PCR primers spanning several target regions within the ORF6 gene were evaluated for specificity to *M. pneumoniae.* Sequence conservation within the selected target region was assessed by sequence analysis of PCR products from 8 reference strains of *M. pneumoniae* to demonstrate homology in the target region. Two SDA systems were designed within the PCR amplified ORF6 target region. Primers developed for use in tSDA are shown in Table 1. Also shown are signal primers and a reporter probe for amplification and detection of the resultant amplicons. The exemplary restriction endonuclease recognition sites (*Bso*Bl) in the amplification primers are shown in boldface type and the target binding sequences are italicized. The target binding sequence of an amplification primer determines its target specificity.

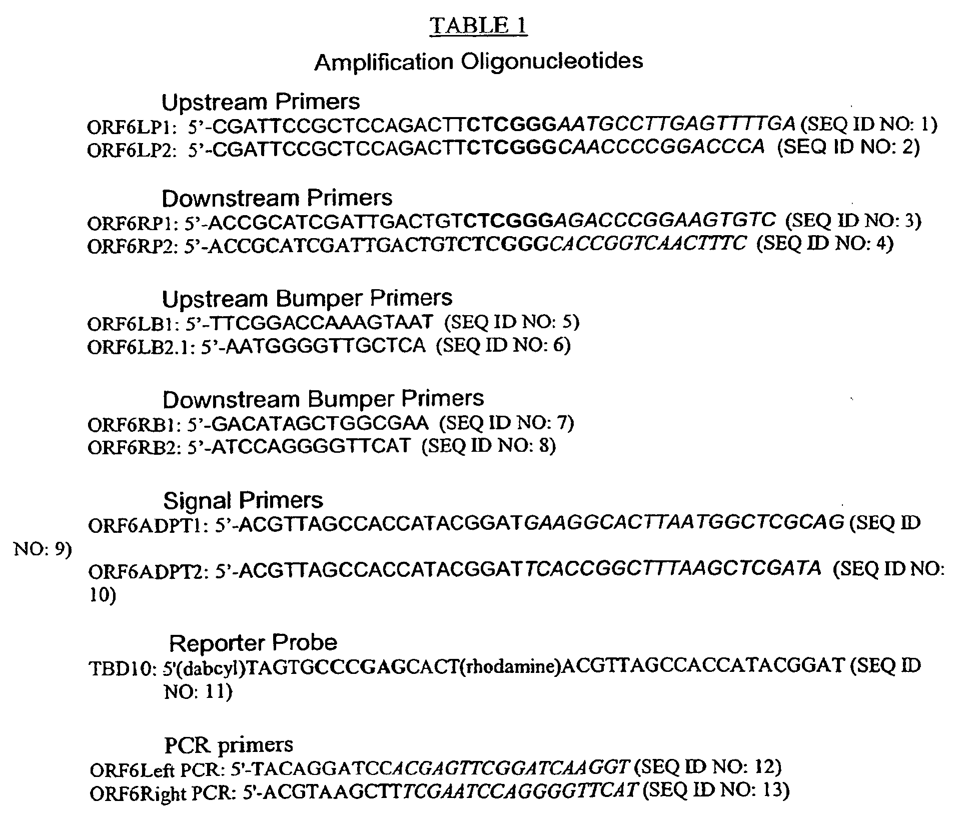

As nucleic acids do not require complete complementarity in order to hybridize, it is to be understood that the probe and primer sequences herein disclosed may be modified to some extent without loss of utility as *M. pneumoniae-*specific probes and primers. As is known in the art, hybridization of complementary and partially complementary nucleic acid sequences may be obtained by adjustment of the hybridization conditions to increase or decrease stringency (i.e., adjustment of hybridization pH, temperature or salt content of the buffer). Such minor modifications of the disclosed sequences and any necessary adjustments of hybridization conditions to maintain *M*. *pneumoniae-specificity* require only routine experimentation and are within the ordinary skill in the art.

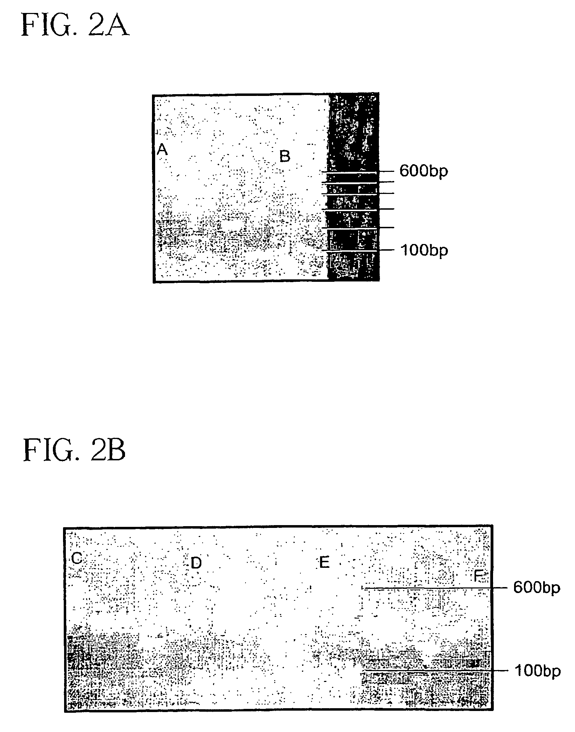

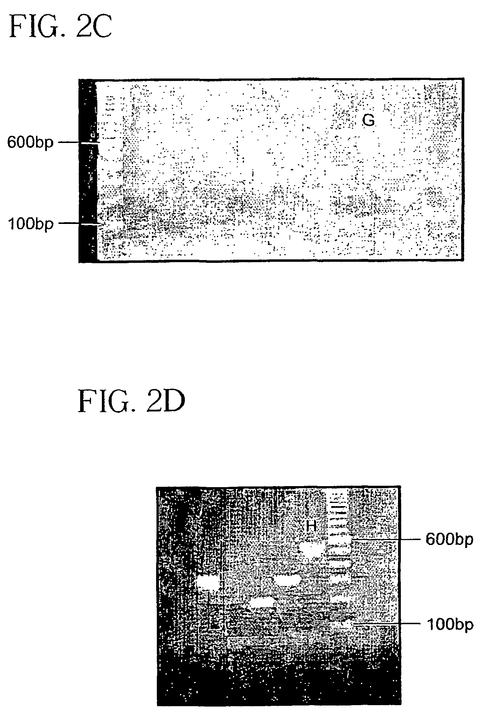

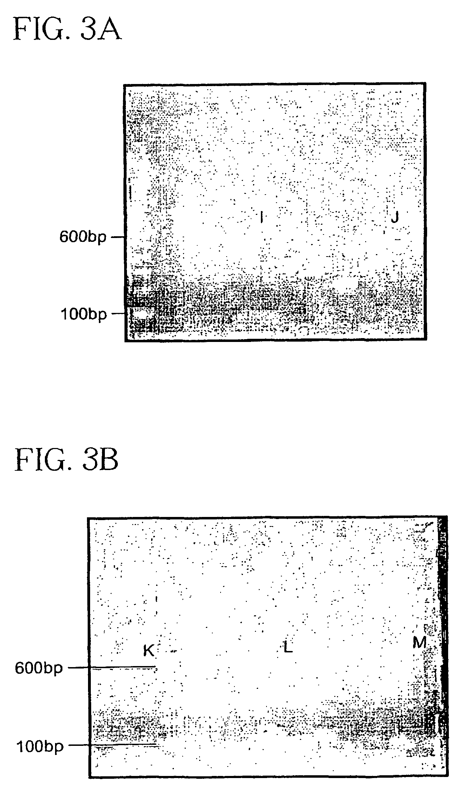

The amplification products generated using the primers disclosed herein may be detected by a characteristic size, for example as illustrated in Fig. 2 and 3, on polyacrylamide or agarose gels stained with ethidium bromide. Alternatively, amplified target sequences may be detected by means of an assay probe, which is an oligonucleotide tagged with a detectable label. In one embodiment, at least one tagged assay probe may be used for detection of amplified target sequences by hybridization (a detector probe), by hybridization and extension as described by Walker, et al. (1992, *Nucl. Acids Res.* 20:1691-1696) (a detector primer) or by hybridization, extension and conversion to double stranded form as described in EP 0 678 582 (a signal primer).

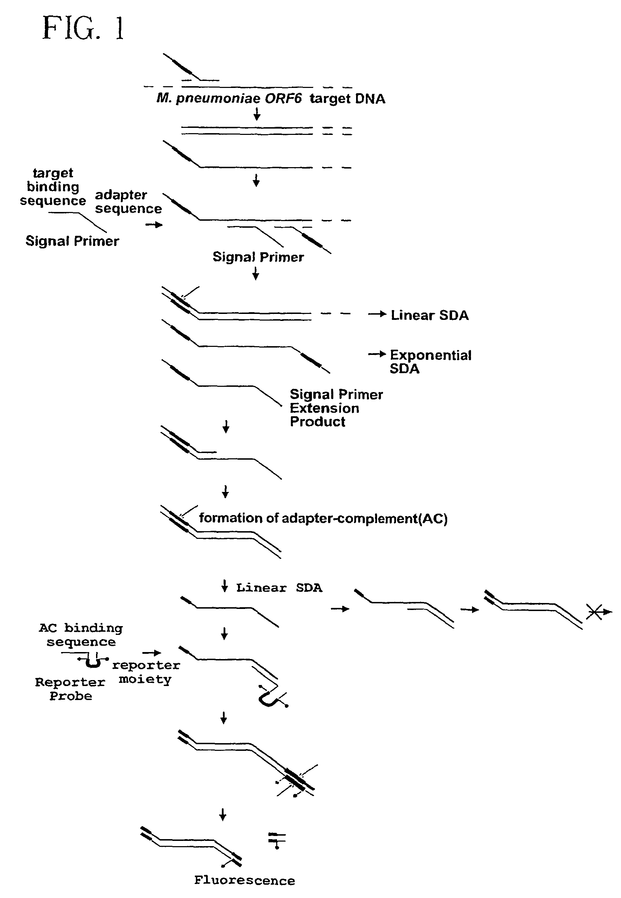

A preferred embodiment for the detection of amplified target is illustrated schematically in Fig. 1. In this embodiment, the 5' tail sequence of the signal primer is comprised of a sequence that does not hybridize to the target (the adapter sequence). The adapter sequence is an indirectly detectable marker that may be selected such that it is the same in a variety of signal primers that have different 3' target binding sequences (i.e., a "universal" 5' tail sequence). Oligonucleotides having SEQ ID NOs: 9 and 10 are particularly useful as signal primers, in conjunction with the amplification primers of the invention for detection of *M*. *pneumoniae* organisms. Preferably, an assay probe is a single reporter probe sequence that hybridizes to the adapter sequence complement of the signal primers of the invention. An oligonucleotide having the sequence of SEQ ID NO: 11 is particularly useful as a reporter probe when used in conjunction with the signal primers of the invention for detection of *M*. *pneumoniae.* Alternatively, an assay probe can be selected to hybridize to a sequence in the target that is between the amplification primers. In a further embodiment, an amplification primer or the target binding sequence thereof may be used as the assay probe.

The detectable label of the assay probe is a moiety that can be detected either directly or indirectly as an indication of the presence of the target nucleic acid. For direct detection of the label, assay probes may be tagged with a radioisotope and detected by autoradiography or tagged with a fluorescent moiety and detected by fluorescence as is known in the art. Alternatively, the assay probes may be indirectly detected by tagging with a label that requires additional reagents to render it detectable. Indirectly detectable labels include, for example, chemiluminescent agents, enzymes that produce visible reaction products and ligands (e.g., haptens, antibodies or antigens) which may be detected by binding to labeled specific binding partners (e.g., antibodies or antigens/haptens). Ligands are also useful for immobilizing the ligand-labeled oligonucleotide (the capture probe) on a solid phase to facilitate its detection. Particularly useful labels include biotin (detectable by binding to labeled avidin or streptavidin) and enzymes such as horseradish peroxidase or alkaline phosphatase (detectable by addition of enzyme substrates to produce colored reaction products). Methods for adding such labels to, or including such labels in, oligonucleotides are well known in the art and any of these methods are suitable for use in the present invention.

Examples of specific detection methods which may be employed include a chemiluminescent method in which amplified products are detected using a biotinylated capture probe and an enzyme-conjugated detector probe as described in U.S. Patent No. 5,470,723. After hybridization of these two assay probes to different sites in the assay region of the target sequence (between the binding sites of the two amplification primers), the complex is captured on a streptavidin-coated microtiter plate by means of the capture probe, and the chemiluminescent signal is developed and read in a luminometer. As another alternative for detection of amplification products, a signal primer as described in EP 0 678 582 may be included in the SDA reaction. In yet another alternative for detection of amplification products, the signal primer may contain sequences that do not hybridize to the target sequence, i.e., the adapter sequence. In this embodiment, as illustrated in Fig.1, a reporter probe with associated label can hybridize to the complement of the adapter sequence. In both embodiments of the signal primer, secondary amplification products are generated during SDA in a target amplification-dependent manner and may be detected as an indication of target amplification.

For commercial convenience, amplification primers for specific detection and identification of nucleic acids may be packaged in the form of a kit. Typically, such a kit contains at least one pair of amplification primers. Reagents for performing a nucleic acid amplification reaction may also be included with the target-specific amplification primers, for example, buffers, additional primers, nucleotide triphosphates, enzymes, etc. The components of the kit are packaged together in a common container, optionally including instructions for performing a specific embodiment of the inventive methods. Other optional components may also be included in the kit, e.g., an oligonucleotide tagged with a label suitable for use as an assay probe, and/or reagents or means for detecting the label.

For the present invention, such a kit may be configured in order to provide the necessary components for a respiratory panel of organisms. Such a respiratory panel may include *Bordetella pertussis, Legionella pneumophila, M. pneumoniae* and organisms of the Chlamydiaceae family in addition to other microorganisms capable of causing respiratory infection. Thus, such a respiratory panel kit would include the primers for amplification of a nucleic acid sequence specific for each of the organisms of the respiratory panel. Useful primers, bumpers, signal primers and reporter probes for amplifying and detecting B. *pertussis, L. pneumophila* and Chlamydiaceae Family organisms are described in co-pending U.S. Patent Application Serial No. 09/626,855, filed on July 27, 2000, co-pending U.S. Patent Application Serial No. 09/626,354, filed on July 27, 2000 and co-pending U.S. Patent Application Serial No. 09/708,208, filed November 8, 2000, respectively. When used, such a respiratory panel kit may permit separate amplification reactions for each organism or one or more multiplex amplification reactions to provide results indicating the presence or absence of each of the organisms of the panel.

The target binding sequences of the amplification primers confer species hybridization specificity on the oligonucleotides and therefore provide species specificity to the amplification reaction. Thus, the target binding sequences of the amplification primers of the invention are also useful in other nucleic acid amplification protocols such as the PCR, conventional SDA (a reaction scheme which is essentially the same as that of tSDA but conducted at lower temperatures using mesophilic enzymes), 3SR, NASBA, TAS and RCA. Specifically, any amplification protocol which utilizes cyclic, specific hybridization of primers to the target sequence, extension of the primers using the target sequence as a template and separation or displacement of the extension products from the target sequence may employ the target binding sequences of the invention. For amplification methods that do not require specialized, non-target binding sequences (e.g., PCR), the amplification primers may consist essentially of the target binding sequences of the amplification primers listed in Table 1.

Other sequences, as required for performance of a selected amplification reaction, may optionally be added to the target binding sequences disclosed herein without altering the species specificity of the oligonucleotide. By way of example, the specific amplification primers may contain a recognition site for the restriction endonuclease BsoBl that is nicked during the SDA reaction. It will be apparent to one skilled in the art that other nickable restriction endonuclease recognition sites may be substituted for the BsoBl recognition site including, but not limited to, those recognition sites disclosed in EP 0 684 315. Preferably, the recognition site is for a thermophilic restriction endonuclease so that the amplification reaction may be performed under the conditions of tSDA. Similarly, the tail sequence of the amplification primer (5' to the restriction endonuclease recognition site) is generally not critical, although the restriction site used for SDA and sequences which will hybridize either to their own target binding sequence or to the other primers should be avoided. Some amplification primers for SDA therefore consist of 3' target binding sequences, a nickable restriction endonuclease recognition site 5' to the target binding sequence and a tail sequence about 10-25 nucleotides in length 5' to the restriction endonuclease recognition site. The nickable restriction endonuclease recognition site and the tail sequence are sequences required for the SDA reaction. As described in U.S. Patent Application Serial No. 09/573,242, filed May 18, 2000, some amplification primers for SDA can consist of target specific sequences both 5' and 3' of the restriction enzyme recognition site. An increase in the efficiency of target specific hybridization may be attained with this design. For other amplification reactions (e.g., 3SR, NASBA, TAS and RCA), the amplification primers may consist of the target binding sequence and additional sequences required for the selected amplification reaction (e.g., sequences required for SDA as described above or a promoter recognized by RNA polymerase for 3SR). Adaptation of the target binding sequences of the invention to amplification methods other than SDA employs routine methods for preparation of amplification primers, such as chemical synthesis, and the well known structural requirements for the primers of the selected amplification reaction. The target binding sequences of the invention may therefore be readily adapted to *M. pneumoniae* organism-specific target amplification and detection in a variety of amplification reactions using only routine methods for production, screening and optimization.

In SDA, the bumper primers are not essential for species specificity, as they function to displace the downstream, species-specific amplification primers. It is required only that the bumper primers hybridize to the target upstream from the amplification primers so that when they are extended they will displace the amplification primer and its extension product. The particular sequence of the bumper primer is therefore generally not critical, and may be derived from any upstream target sequences which are sufficiently close to the binding site of the amplification primer to allow displacement of the amplification primer extension product upon extension of the bumper primer. Occasional mismatches with the target in the bumper primer sequence or some cross-hybridization with non-target sequences do not generally negatively affect amplification efficiency as long as the bumper primer remains capable of hybridizing to the specific target sequence.

Amplification reactions employing the primers of the invention may incorporate thymine as taught by Walker, et al. (1992, *Nucl. Acids Res.* 20:1691-1696), or they may wholly or partially substitute 2'-deoxyuridine 5'-triphosphate for TTP in the reaction to reduce cross-contamination of subsequent amplification reactions, e.g., as taught in EP 0 624 643. dU (uridine) is incorporated into amplification products and can be excised by treatment with uracil DNA glycosylase (UDG). These abasic sites render the amplification product unamplifiable in subsequent amplification reactions. UDG may be inactivated by uracil DNA glycosylase inhibitor (UGI) prior to performing the subsequent amplification to prevent excision of dU in newly-formed amplification products.

SDA is an isothermal method of nucleic acid amplification in which extension of primers, nicking of a hemimodified restriction endonuclease recognition/cleavage site, displacement of single stranded extension products, annealing of primers to the extension products (or the original target sequence) and subsequent extension of the primers occurs concurrently in the reaction mix. This is in contrast to PCR, in which the steps of the reaction occur in discrete phases or cycles as a result of the temperature cycling characteristics of the reaction. SDA is based upon 1) the ability of a restriction endonuclease to nick the unmodified strand of a hemiphosphorothioate form of its double stranded recognition/cleavage site and 2) the ability of certain polymerases to initiate replication at the nick and displace the downstream non-template strand. After an initial incubation at increased temperature (about 95°C) to denature double stranded target sequences for annealing of the primers, subsequent polymerization and displacement of newly synthesized strands takes place at a constant temperature. Production of each new copy of the target sequence consists of five steps: 1) binding of amplification primers to an original target sequence or a displaced single-stranded extension product previously polymerized, 2) extension of the primers by a 5'-3' exonuclease deficient polymerase incorporating an α-thio deoxynucleoside triphosphate (α-thio dNTP), 3) nicking of a hemimodified double stranded restriction site, 4) dissociation of the restriction enzyme from the nick site, and 5) extension from the 3' end of the nick by the 5'-3' exonuclease deficient polymerase with displacement of the downstream newly synthesized strand. Nicking, polymerization and displacement occur concurrently and continuously at a constant temperature because extension from the nick regenerates another nickable restriction site. When a pair of amplification primers is used, each of which hybridizes to one of the two strands of a double stranded target sequence, amplification is exponential. This is because the sense and antisense strands serve as templates for the opposite primer in subsequent rounds of amplification. When a single amplification primer is used, amplification is linear because only one strand serves as a template for primer extension. Examples of restriction endonucleases which nick their double stranded recognition/cleavage sites when an α-thio dNTP is incorporated are *Hin*cII*, Hin*dII*, Ava*I*, Nci*I and *Fnu*4HI*.* All of these restriction endonucleases and others that display the required nicking activity are suitable for use in conventional SDA. However, they are relatively thermolabile and lose activity above about 40°C.

Targets for amplification by SDA may be prepared by fragmenting larger nucleic acids by restriction with an endonuclease that does not cut the target sequence. However, it is generally preferred that target nucleic acids having selected restriction endonuclease recognition/cleavage sites for nicking in the SDA reaction be generated as described by Walker, et al. (1992, *Nucl. Acids Res.* 20:1691-1696) and in U.S. Patent No. 5,270,184 (specifically incorporated herein by reference). Briefly, if the target sequence is double stranded, four primers are hybridized to it. Two of the primers (S₁ and S₂) are SDA amplification primers and two (B₁ and B₂) are external or bumper primers. S₁ and S₂ bind to opposite strands of double stranded nucleic acids flanking the target sequence. B₁ and B₂ bind to the target sequence 5' (i.e., upstream) of S₁ and S₂, respectively. The exonuclease deficient polymerase is then used to simultaneously extend all four primers in the presence of three deoxynucleoside triphosphates and at least one modified deoxynucleoside triphosphate (e.g., 2'-deoxyadenosine 5'-O-(1-thiotriphosphate), "dATPαS"). The extension products of S₁ and S₂ are thereby displaced from the original target sequence template by extension of B₁ and B₂. The displaced, single stranded extension products of the amplification primers serve as targets for binding of the opposite amplification and bumper primer (e.g., the extension product of S₁ binds S₂ and B₂). The next iteration of extension and displacement results in two double stranded nucleic acid fragments with hemimodified restriction endonuclease recognition/cleavage sites at each end. These are suitable substrates for amplification by SDA. As in SDA, the individual steps of the target generation reaction occur concurrently and continuously, generating target sequences with the recognition/cleavage sequences at the ends required for nicking by the restriction enzyme in SDA. As all of the components of the SDA reaction are already present in the target generation reaction, target sequences generated automatically and continuously enter the SDA iteration and are amplified.

To prevent cross-contamination of one SDA reaction by the amplification products of another, dUTP may be incorporated into SDA-amplified DNA in place of dTTP without inhibition of the amplification reaction. The uracil-modified nucleic acids may then be specifically recognized and inactivated by treatment with uracil DNA glycosylase (UDG). Therefore, if dUTP is incorporated into SDA-amplified DNA in a prior reaction, any subsequent SDA reactions can be treated with UDG prior to amplification of double stranded targets, and any dU containing DNA from previously amplified reactions will be rendered unamplifiable. The target DNA to be amplified in the subsequent reaction does not contain dU and will not be affected by the UDG treatment. UDG may then be inhibited by treatment with UGI prior to amplification of the target. Alternatively, UDG may be heat-inactivated. In tSDA, the higher temperature of the reaction itself (≥ 50°C) can be used concurrently to inactivate UDG and amplify the target.

SDA requires a polymerase which lacks 5'-3' exonuclease activity, initiates polymerization at a single stranded nick in double stranded nucleic acids, and displaces the strand downstream of the nick while generating a new complementary strand using the unnicked strand as a template. The polymerase must extend by adding nucleotides to a free 3'-OH. To optimize the SDA reaction, it is also desirable that the polymerase be highly processive to maximize the length of target sequence that can be amplified. Highly processive polymerases are capable of polymerizing new strands of significant length before dissociating and terminating synthesis of the extension product. Displacement activity is essential to the amplification reaction, as it makes the target available for synthesis of additional copies and generates the single stranded extension product to which a second amplification primer may hybridize in exponential amplification reactions. Nicking activity of the restriction enzyme is also of great importance, as it is nicking which perpetuates the reaction and allows subsequent rounds of target amplification to initiate.

tSDA is performed essentially as the conventional SDA described by Walker, et al. (1992, *Proc. Natl. Acad. Sci. USA* 89:392-396 and 1992, *Nucl. Acids Res.* 20:1691-1696), with substitution of a polymerase and a restriction endonuclease that remain active at higher temperatures than the enzymes used in SDA. Of course, the temperature of the reaction will be adjusted to the higher temperature suitable for the substituted enzymes and the *Hinc*II restriction endonuclease recognition/cleavage site will be replaced by the appropriate restriction endonuclease recognition/cleavage site for the selected endonuclease. Also in contrast to Walker, et al., the practitioner may include the enzymes in the reaction mixture prior to the initial denaturation step if they are sufficiently stable at the denaturation temperature. Preferred restriction endonucleases for use in tSDA are *Bsr*l, *Bst*Nl, *Bsm*Al, *Bsl*I and *Bso*Bl (New England BioLabs), and *Bst*Ol (Promega). The preferred polymerases for tSDA are *Bca* (Panvera) and *Bst* (New England Biolabs).

Further, SDA and tSDA reactions for amplifying longer nucleic acid targets (e.g. nucleic acid molecules greater than about 100 base pairs in length) may be enhanced by including a DNA binding protein, such as gp32, in the reaction mixture. This technique is known to those skilled in the art through its thorough description in European Patent Application Publication No. 0 869 187 A2.

Homogeneous real time fluorescent tSDA is a modification of tSDA. It employs detector oligonucleotides to produce reduced fluorescence quenching in a target-dependent manner. The detector oligonucleotides contain a donor/acceptor dye pair linked such that fluorescence quenching occurs in the absence of target. Unfolding or linearization of an intramolecularly base-paired secondary structure in the detector oligonucleotide in the presence of the target increases the distance between the dyes and reduces fluorescence quenching. Unfolding of the base-paired secondary structure typically involves intermolecular base-pairing between the sequence of the secondary structure and a complementary strand such that the secondary structure is at least partially disrupted. It may be fully linearized in the presence of a complementary strand of sufficient length. In a preferred embodiment, a restriction endonuclease recognition site (RERS) is present between the two dyes such that intermolecular base-pairing between the secondary structure and a complementary strand also renders the RERS double-stranded and cleavable by a restriction endonuclease. Cleavage by the restriction endonuclease separates the donor and acceptor dyes onto separate nucleic acid fragments, further contributing to decreased quenching. In either embodiment, an associated change in a fluorescence parameter (e.g., an increase in donor fluorescence intensity, a decrease in acceptor fluorescence intensity or a ratio of fluorescence before and after unfolding) is monitored as an indication of the presence of the target sequence. Monitoring a change in donor fluorescence intensity is preferred, as this change is typically larger than the change in acceptor fluorescence intensity. Other fluorescence parameters such as a change in fluorescence lifetime may also be monitored. Cleavage of an oligonucleotide refers to breaking the phosphodiester bonds of both strands of a DNA duplex or breaking the phosphodiester bond of single-stranded DNA. This is in contrast to nicking, which refers to breaking the phosphodiester bond of only one of the two strands in a DNA duplex.

A detector oligonucleotide for homogeneous real time fluorescent tSDA may be an oligonucleotide which comprises both a single-stranded 5' or 3' section which hybridizes to the target sequence (the target binding sequence), as well as an intramolecularly base-paired secondary structure adjacent to the target binding sequence. In a preferred embodiment, as illustrated in Fig. 1, the detector oligonucleotide is a reporter probe that comprises a single-stranded 5' or 3' section that does not hybridize to the target sequence. Rather, the single-stranded 5' or 3' section hybridizes to the complement of the signal primer adapter sequence (the adapter-complement binding sequence). A further characteristic of the reporter probe is that this hybridizing section is adjacent to an intramolecularly base-paired secondary structure. The detector oligonucleotides of the invention further comprise a donor/acceptor dye pair linked to the detector oligonucleotide such that donor fluorescence is quenched when the secondary structure is intramolecularly base-paired and unfolding or linearization of the secondary structure results in a decrease in fluorescence quenching.

The detector oligonucleotides of the invention for homogeneous real time fluorescent tSDA comprise a sequence that forms an intramolecularly base-paired secondary structure under the selected reaction conditions for primer extension or hybridization. In one embodiment, the secondary structure may be positioned adjacent to the target binding sequence of the detector oligonucleotide so that at least a portion of the target binding sequence forms a single-stranded 3' or 5' tail. In a preferred embodiment, as illustrated in Fig. 1, the secondary structure is positioned adjacent to the adapter-complement binding sequence of the reporter probe detector oligonucleotide so that at least a portion of the adapter-complement binding sequence forms a single-stranded 3' or 5' tail. As used herein, the term "adjacent to the target binding sequence" or "adjacent to the adapter-complement binding sequence" means that all or part of the target/adapter-complement binding sequence is left single-stranded in a 5' or 3' tail which is available for hybridization to the target/adapter-complement. That is, the secondary structure does not comprise the entire target/adapter-complement binding sequence. A portion of the target/adapter-complement binding sequence may be involved in the intramolecular base-pairing in the secondary structure, it may include all or part of a first sequence involved in intramolecular base-pairing in the secondary structure but preferably does not extend into its complementary sequence. For example, if the secondary structure is a stem-loop structure (e.g., a "hairpin") and the target/adapter-complement binding sequence of the detector oligonucleotide is present as a single-stranded 3' tail, the target/adapter-complement binding sequence may also extend through all or part of the first arm of the stem and, optionally, through all or part of the loop. However, the target/adapter-complement binding sequence preferably does not extend into the second arm of the sequence involved in stem intramolecular base-pairing. That is, it is desirable to avoid having both sequences involved in intramolecular base-pairing in a secondary structure capable of hybridizing to the target/adapter-complement. Mismatches in the intramolecularly base-paired portion of the detector oligonucleotide secondary structure may reduce the magnitude of the change in fluorescence in the presence of target but are acceptable if assay sensitivity is not a concern. Mismatches in the target/adapter-complement binding sequence of the single-stranded tail are also acceptable but may similarly reduce assay sensitivity and/or specificity. However, it is a feature of the present invention that perfect base-pairing in both the secondary structure and the target/adapter-complement binding sequence do not compromise the reaction. Perfect matches in the sequences involved in hybridization improve assay specificity without negative effects on reaction kinetics.

When added to the amplification reaction, the detector oligonucleotide reporter probe of the invention is converted to double-stranded form by hybridization and extension as illustrated in Fig. 1. Strand displacement by the polymerase also unfolds or linearizes the secondary structure and converts it to double-stranded form by synthesis of a complementary strand. The RERS, if present, also becomes double-stranded and cleavable by the restriction endonuclease. As the secondary structure is unfolded or linearized by the strand displacing activity of the polymerase, the distance between the donor and acceptor dye is increased, thereby reducing quenching of donor fluorescence. The associated change in fluorescence of either the donor or acceptor dye may be monitored or detected as an indication of amplification of the target sequence. Cleavage of the RERS generally further increases the magnitude of the change in fluorescence by producing two separate fragments of the double-stranded secondary amplification product, each having one of the two dyes linked to it. These fragments are free to diffuse in the reaction solution, further increasing the distance between the dyes of the donor/acceptor pair. An increase in donor fluorescence intensity or a decrease in acceptor fluorescence intensity may be detected and/or monitored as an indication that target amplification is occurring or has occurred, but other fluorescence parameters which are affected by the proximity of the donor/acceptor dye pair may also be monitored. A change in fluorescence intensity of the donor or acceptor may also be detected as a change in a ratio of donor and/or acceptor fluorescence intensities. For example, a change in fluorescence intensity may be detected as; a) an increase in the ratio of donor fluorophore fluorescence after linearizing or unfolding the secondary structure and donor fluorophore fluorescence in the detector oligonucleotide prior to linearizing or unfolding, or b) as a decrease in the ratio of acceptor dye fluorescence after linearizing or unfolding and acceptor dye fluorescence in the detector oligonucleotide prior to linearizing or unfolding.

It will be apparent that, in addition to SDA, the detector oligonucleotides of the invention may be adapted for use in the detection of amplicons in other primer extension amplification methods (e.g., PCR, 3SR, TAS or NASBA). For example, the methods may be adapted for use in PCR by using PCR amplification primers and a strand displacing DNA polymerase which lacks 5'→3' exonuclease activity (e.g., Sequencing Grade Taq from Promega or exo Vent or exo Deep Vent from New England BioLabs) in the PCR. The signal primers hybridize to the target at least partially downstream from the PCR amplification primers, are displaced and are rendered double-stranded after hybridization to the detector oligonucleotide reporter probe and subsequent extension. In PCR any RERS may optionally be selected for use in the detector oligonucleotide, as there are typically no modified deoxynucleoside triphosphates present which might induce nicking rather than cleavage of the RERS. As thermocycling is a feature of amplification by PCR, the restriction endonuclease is preferably added at low temperature after the final cycle of primer annealing and extension for end-point detection of amplification. However, a thermophilic restriction endonuclease that remains active through the high temperature phases of the PCR reaction could be present during amplification to provide a real-time assay. As in SDA systems, separation of the dye pair reduces fluorescence quenching, with a change in a fluorescence parameter such as intensity serving as an indication of target amplification.

The change in fluorescence resulting from unfolding or linearizing of the detector oligonucleotides may be detected at a selected endpoint in the reaction. However, because linearized secondary structures are produced concurrently with hybridization or primer extension, the change in fluorescence may also be monitored as the reaction is occurring, i.e., in "real-time". This homogeneous, real-time assay format may be used to provide semiquantitative or quantitative information about the initial amount of target present. For example, the rate at which fluorescence intensity changes during the unfolding or linearizing reaction (either as part of target amplification or in non-amplification detection methods) is an indication of initial target levels. As a result, when more initial copies of the target sequence are present, donor fluorescence more rapidly reaches a selected threshold value (i.e., shorter time to positivity). The decrease in acceptor fluorescence similarly exhibits a shorter time to positivity, detected as the time required to reach a selected minimum value. In addition, the rate of change in fluorescence parameters during the course of the reaction is more rapid in samples containing higher initial amounts of target than in samples containing lower initial amounts of target (i.e., increased slope of the fluorescence curve). These or other measurements as are known in the art (e.g., U.S. Patent No. 5,928,907, U.S. Patent Application Serial No. 09/196,123, filed November 20, 1998, and U.S. Patent Application Serial No. 09/574,031, filed May, 19, 2000, all of which are specifically incorporated by reference herein) may be made as an indication of the presence of target or as an indication of target amplification. The initial amount of target is typically determined by comparison of the experimental results to results for known amounts of target.

Assays for the presence of a selected target sequence according to the methods of the invention may be performed in solution or on a solid phase. Real-time or endpoint homogeneous assays in which the detector oligonucleotide functions as a primer are typically performed in solution. Hybridization assays using the detector oligonucleotides of the invention may also be performed in solution (e.g., as homogeneous real-time assays) but are also particularly well-suited to solid phase assays for real-time or endpoint detection of target. In a solid phase assay, detector oligonucleotides may be immobilized on the solid phase (e.g., beads, membranes or the reaction vessel) via internal or terminal labels using methods known in the art. For example, a biotin-labeled detector oligonucleotide may be immobilized on an avidin-modified solid phase where it will produce a change in fluorescence when exposed to the target under appropriate hybridization conditions. Capture of the target in this manner facilitates separation of the target from the sample and allows removal of substances in the sample that may interfere with detection of the signal or other aspects of the assay. An example of a solid phase system that can be used is an array format, such as those known in the art.

### EXAMPLES

The following Examples illustrate specific embodiments of the invention described herein. As would be apparent to skilled artisans, various changes and modifications are possible, and are contemplated within the scope of the invention described.

### EXAMPLE 1

### ORF6 M. pneumoniae Specificity

In order to determine the specificity of the ORF6 gene for *M. pneumoniae,* PCR amplification was performed with DNA from several *M. pneumoniae* strains using primers SEQ ID NO: 12 and SEQ ID NO: 13. In brief, PCR conditions were as follows: 10 µM each of primers SEQ ID NO: 12 and SEQ ID NO: 13; 200 nM each of dATP, dTTP, dCTP and dGTP; 5 units of AmpliTAQ DNA polymerase (Perkin Elmer), and 1 µl of 1:10 dilution of ATCC organism stock in a 50 µl reaction volume containing 1x AmpliTAQ buffer. PCR was carried out on a MJ Research PTC-200 Peltier Thermal Cycler and temperature cycling conditions consisted of one cycle at 95°C for 5 minutes followed by 35 cycles at 95°C for 45 seconds, at 52°C for 45 seconds, and at 72°C for 60 seconds. Nine microliters of each PCR product were mixed with 1 µl loading dye and analyzed by agarose gel electrophoresis. A 100 bp ladder (GibcoBRL) gel marker was used to estimate product size. The gel was run at 98 volts for approximately 45 minutes. Figure 2 illustrates the results of 1% agarose gel analysis of products from PCR amplification of 8 *M. pneumoniae* ATCC strains. Table 1 shows the strains tested with the identification of bands corresponding to labeled bands in Fig. 2.

| Table 1 | |
|---|---|
| **Band ID** | **ATCC strain** |
| A | 15531 |
| B | 15492 |
| C | 15293 |
| D | 15377 |
| E | 29085 |
| F | 39505 |
| G | 29342 |
| H | 49894 |

In each case, a PCR product of 529 bp (ORF6 amplified target region) was observed, demonstrating the presence of the ORF6 gene in all eight *M. pneumoniae* strains.

In order to determine whether homologues of the ORF6 gene exist in species that are phylogenetically related to *M*. *pneumoniae,* DNA from 4 other species of *Mycoplasma* was amplified by PCR using primers SEQ ID NO: 12 and SEQ ID NO: 13 under the conditions described above. PCR products were analyzed by electrophoresis on a 1.5% agarose gel as illustrated in Fig. 3. The results of this analysis are summarized in Table 2. Only the *M. pneumoniae* positive control (ATCC 29342) yielded a band of the predicted size of 529 bp, indicating that the ORF6 primers SEQ ID NO: 12 and SEQ ID NO: 13 are specific for this species.

| Table 2 | |
|---|---|
| **Band ID** | **Organism/strain** |
| I | *M. orale*/ATCC strain 15539 |
| J | *M. pneumoniae*/*ATCC* strain 29342 (control) |
| K | *M. gallisepticuml* ATCC strain 19610 |
| L | *M. synoviae*/ATCC strain 25204 |
| M | *M. genitaliumlATCC* strain 33530 |

### EXAMPLE 2

### Sequence Alignments

The PCR products generated in Example 1 were sequenced and an alignment of the eight ORF6 sequences was compiled using the MegAlign software program (DNAStar).

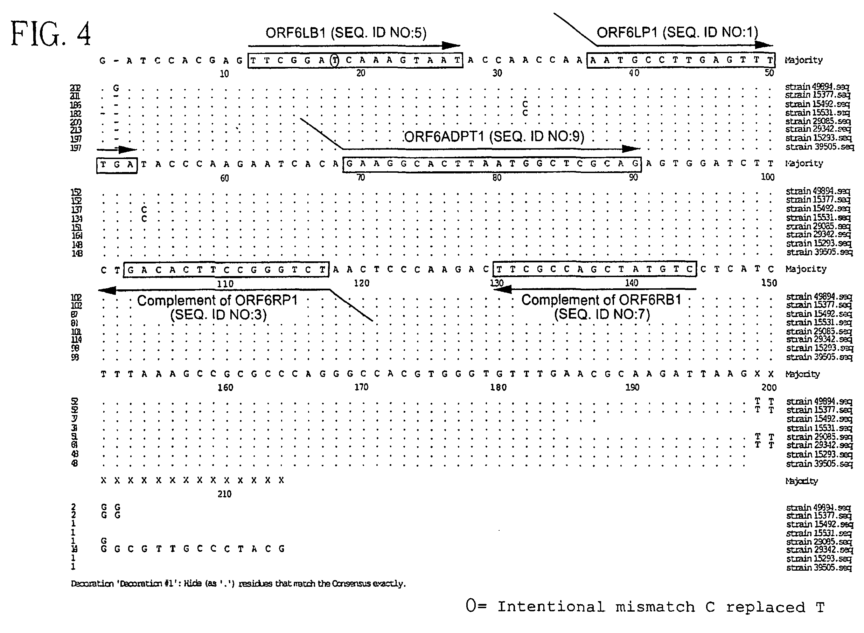

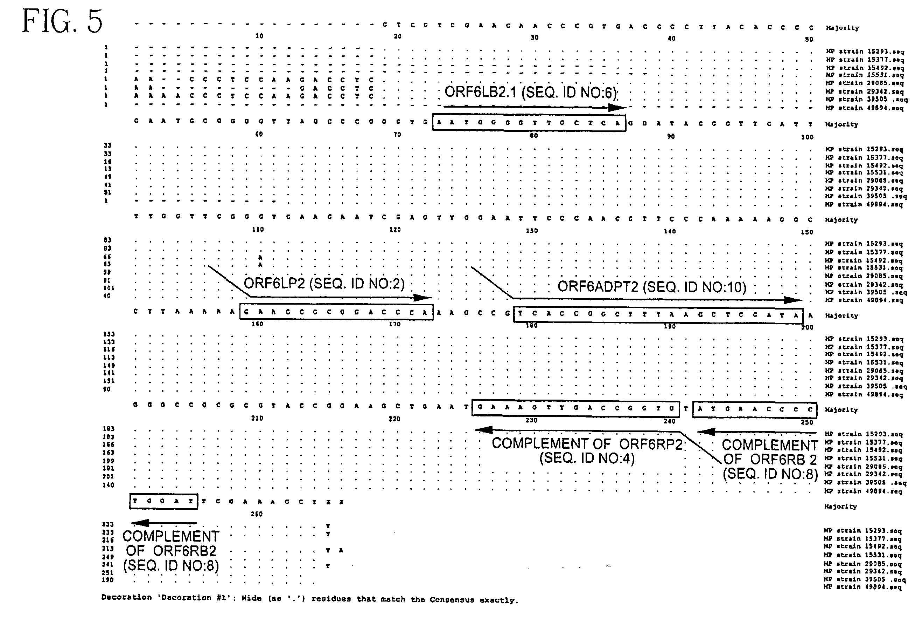

Two SDA systems were designed within the ORF6 region bounded by primers SEQ ID NO: 12 and SEQ ID NO: 13. Fig. 4 illustrates the alignment of the target binding sequences of SDA system 1 oligonucleotides SEQ ID NO: 1, SEQ ID NO: 3, SEQ ID NO: 5, SEQ ID NO: 7 and SEQ ID NO: 9 to the ORF6 target region. An intentional mismatch was incorporated into SEQ ID NO: 5 (C replaced by T) to avoid interactions with SEQ ID NO: 1. Fig. 5 illustrates the alignment of the target binding sequences of SDA system 2 oligonucleotides SEQ ID NO: 2, SEQ ID NO: 4, SEQ ID NO: 6, SEQ ID NO: 8 and SEQ ID NO: 10 to the ORF6 target region. No mismatches are present within the target binding regions of these oligonucleotides.

The alignments demonstrate homology within the oligonucleotide target binding sequences for both ORF6 SDA systems across all strains of *M. pneumoniae* evaluated.

### EXAMPLE 3

### SDA Systems Designed for the ORF6 gene

In order to assess specificity of the oligonucleotides comprising the two aforementioned SDA systems for the ORF6 target sequence, nucleic acid sequence homologies were evaluated using the Basic Local Alignment Search Tool ("BLAST") which is well known in the art (1990, *Proc. Natl. Acad.* Sci. USA 87:2267-2268; 1990, *J. Mol. Biol.* 215:403-410; 1993, *Nature Genetics* 3:266-272; 1997, *Nuc. Acids Res.* 25:3389-3402). BLASTN was used to compare a nucleotide query sequence against a nucleotide sequence database. The BLASTN program identifies homologous sequences by identifying similar segments, which are referred to herein as "high-scoring segment pairs," between a query nucleic acid sequence and a test sequence which is preferably obtained from a nucleic acid sequence database. High-scoring segment pairs are preferably identified (ie. aligned) by means of a scoring matrix, many of which are known in the art. Preferably, the scoring matrix used is the Blosum62 matrix (1992, *Science* 256:1443-1445; 1993, *Proteins* 17:49-61). The BLASTN program evaluates the statistical significance of all high-scoring segment pairs identified, and preferably selects those segments which satisfy a user-specified threshold of significance, such as percent homology. Preferably, the statistical significance of a high-scoring segment pair is evaluated using the statistical significance formula of Karlin (1990, *Proc. Natl. Acad.* Sci. USA 87:2267-2268).

BLASTN analysis was performed on the GenBank database using the target sequence spanning the left and right bumpers for each of ORF6 SDA systems 1 (SEQ ID NO: 5 to SEQ ID NO: 7) and 2 (SEQ ID NO: 6 to SEQ ID NO: 8). Analysis of results only showed significant homology with sequences from the genome of *M*. *pneumoniae.* These results demonstrate that the oligonucleotides comprising ORF6 SDA systems 1 and 2 are specific for *M*. *pneumoniae.*

## Claims

1. An oligonucleotide consisting of a target binding sequence selected from the group consisting of the target binding sequences of ORF6LP1 (SEQ ID NO: 1), ORF6LP2 (SEQ ID NO: 2), ORF6Left PCR (SEQ ID NO: 12), ORF6RP1 (SEQ ID NO: 3), ORF6RP2 (SEQ ID NO: 4) and ORF6Right PCR (SEQ ID NO: 13), and optionally, a sequence required for an amplification reaction.

2. The oligonucleotide of claim 1 wherein the sequence required for the amplification reaction is a restriction endonuclease recognition site that is nickable by a restriction endonuclease.

3. An oligonucleotide selected from the group consisting of ORF6ADPT1 (SEQ ID NO: 9), a nucleic acid complementary to SEQ ID NO: 9, ORF6ADPT2 (SEQ ID NO: 10) and a nucleic acid complementary to SEQ ID NO: 10.

4. A pair of amplification primers comprising:
a) a first primer consisting of a target binding sequence selected from the group consisting of ORF6LP1 (SEQ ID NO: 1), ORF6LP2 (SEQ ID NO: 2) and ORF6Left PCR (SEQ ID NO: 12), and, optionally, a sequence required for an amplification reaction, and;
b) a second primer consisting of a target binding sequence selected from the group consisting of the target binding sequences of ORF6RP1 (SEQ ID NO: 3), ORF6RP2 (SEQ ID NO: 4) and ORF6Right PCR (SEQ ID NO: 13), and, optionally, a sequence required for an amplification reaction.

5. A kit comprising:
a) one or more primers selected from the group consisting of ORF6LP1 (SEQ ID NO: 1); ORF6LP2 (SEQ ID NO: 2) and ORF6Left PCR (SEQ ID NO: 12),
b) one or more primers selected from the group consisting of ORF6RP1 (SEQ ID NO: 3), ORF6RP2 (SEQ ID NO: 4) and ORF6Right PCR (SEQ ID NO: 13).
c) one or more signal primers selected from the group consisting of ORF6ADPT1 (SEQ ID NO: 9), a nucleic acid complementary to SEQ ID NO: 9, ORF6ADPT2 (SEQ ID NO: 10) and a nucleic acid complementary to SEQ ID NO: 10.

6. The kit of claim 5 further comprising:
e) a pair of primers specific for the amplification of a nucleic acid sequence specific for *Legionella pneumophila;*
f) a pair of primers specific for the amplification of a nucleic acid sequence specific for *Bordetella pertussis;* and
g) a pair of primers specific for the amplification of a nucleic acid sequence indicative of a chlamydial infection.

7. A method for detecting the presence or absence of *Mycoplasma pneumoniae* organisms in a sample, said method comprising:
a) treating said sample using a pair of nucleic acid primers in a nucleic acid amplification reaction wherein a said first primer is ORF6LP1 (SEQ ID NO: 1) and a said second primer is ORF6RP1 (SEQ ID NO: 3), and
b) detecting any amplified nucleic acid product, wherein detection of amplified product indicates presence of *Mycoplasma pneumoniae* organisms.

8. A method for detecting the presence or absence of *Mycoplasma pneumoniae* organisms in a sample, said method comprising:
a) treating said sample using a pair of nucleic acid primers in a nucleic acid amplification reaction wherein a said first primer is ORF6LP2. (SEQ ID NO: 2) a said second primer is ORF6RP2 (SEQ ID NO: 4), and
b) detecting any amplified nucleic acid product, wherein detection of amplified product indicates presence of *Mycoplasma pneumoniae* organisms.

9. A method for amplifying a target nucleic acid sequence of a *Mycoplasma pneumoniae* organism comprising:
a) hybridizing to the nucleic acid
i) a first amplification primer selected from the group consisting of the target binding sequences of ORF6LP1 (SEQ ID NO: 1), ORF6LP2 (SEQ ID NO: 2) and ORF6Left PCR (SEQ ID NO: 12), and optionally, a sequence required for an amplification reaction, and
ii) a second amplification primer selected from the group consisting of the target binding sequences of ORF6RP1 (SEQ ID NO: 3), ORF6RP2 (SEQ ID NO: 4) and ORF6Right PCR (SEQ ID NO: 13), and, optionally, a sequence required for the amplification reaction, and;
b) extending the hybridized first and second amplification primers on the target nucleic acid sequence whereby the target nucleic acid sequence is amplified.

10. The method of claim 7, 8 or 9 wherein the nucleic acid amplification reaction is a Polymerase Chain Reaction (PCR) or a Strand Displacement Amplification (SDA) reaction.

## Patentansprüche

1. Oligonucleotid, bestehend aus einer Zielbindungssequenz, die aus der Gruppe ausgewählt ist, die aus den Zielbindungssequenzen von ORF6LP1 (SEQ ID Nr. 1), ORF6LP2 (SEQ ID Nr. 2), ORF6Left PCR (SEQ ID Nr. 12), ORF6RP1 (SEQ ID Nr. 3), ORF6RP2 (SEQ ID Nr. 4) und ORF6Right PCR (SEQ ID Nr. 13) besteht, und gegebenenfalls einer Sequenz, die für eine Amplifikationsreaktion erforderlich ist.

2. Oligonucleotid gemäß Anspruch 1, wobei die für die Amplifikationsreaktion erforderliche Sequenz eine Erkennungsstelle für eine Restriktions-Endonuclease ist, an der von einer Restriktions-Endonuclease ein Einzelstrangbruch eingeführt werden kann.

3. Oligonucleotid, ausgewählt aus der Gruppe, die aus ORF6ADPT1 (SEQ ID Nr. 9), einer zu SEQ ID Nr. 9 komplementären-Nucleinsäure, ORF6ADPT2 (SEQ ID Nr. 10) und einer zu SEQ ID Nr. 10 komplementären Nucleinsäure besteht.

4. Paar von Amplifikationsprimern, umfassend:
a) einen ersten Primer, der aus einer Zielbindungssequenz, die aus der Gruppe ausgewählt ist, die aus ORF6LP1 (SEQ ID Nr. 1), ORF6LP2 (SEQ ID Nr. 2) und ORF6Left PCR (SEQ ID Nr. 12) besteht, und gegebenenfalls einer Sequenz, die für eine Amplifikationsreaktion erforderlich ist, besteht; und
b) einen zweiten Primer, der aus einer Zielbindungssequenz, die aus der Gruppe ausgewählt ist, die aus den Zielbindungssequenzen von ORF6RP1 (SEQ ID Nr. 3), ORF6RP2 (SEQ ID Nr. 4) und ORF6Right PCR (SEQ ID Nr. 13) besteht, und gegebenenfalls einer Sequenz, die für eine Amplifikationsreaktion erforderlich ist, besteht.

5. Kit, umfassend:
a) einen oder mehrere Primer, die aus der Gruppe ausgewählt sind, die aus ORF6LP1 (SEQ ID Nr. 1), ORF6LP2 (SEQ ID Nr. 2) und ORF6Left PCR (SEQ ID Nr. 12) besteht;
b) einen oder mehrere Primer, die aus der Gruppe ausgewählt sind, die aus ORF6RP1 (SEQ ID Nr. 3), ORF6RP2 (SEQ ID Nr. 4) und ORF6Right PCR (SEQ ID Nr. 13) besteht;
c) einen oder mehrere Signalprimer, die aus der Gruppe ausgewählt sind, die aus ORF6ADPT1 (SEQ ID Nr. 9), einer zu SEQ ID Nr. 9 komplementären. Nucleinsäure, ORF6ADPT2 (SEQ ID Nr. 10) und einer zu SEQ ID Nr. 10 komplementären Nucleinsäure besteht.

6. Kit gemäß Anspruch 5, der weiterhin Folgendes umfasst:
e) ein Paar von Primern, die spezifisch für die Amplifikation einer für *Legionella pneumophila* spezifischen Nucleinsäuresequenz sind;
f) ein Paar von Primern, die spezifisch für die Amplifikation einer für *Bordetella pertussis* spezifischen Nucleinsäuresequenz sind; und
g) ein Paar von Primern, die spezifisch für die Amplifikation einer Nucleinsäuresequenz sind, die eine Chlamydieninfektion anzeigt.

7. Verfahren zum Nachweis der Anwesenheit oder Abwesenheit von *Mycoplasma-pneumoniae-Organismen* in einer Probe, wobei das Verfahren Folgendes umfasst:
a) Behandeln der Probe unter Verwendung eines Paars von Nucleinsäureprimern in einer Nucleinsäureamplifikationsreaktion, wobei es sich bei dem ersten Primer um ORF6LP1 (SEQ ID Nr. 1) und bei dem zweiten Primer um ORF6RP1 (SEQ ID Nr. 3) handelt; und
b) Nachweisen eines gegebenenfalls amplifizierten Nucleinsäureprodukts, wobei der Nachweis des amplifizierten Produkts die Anwesenheit von *Mycoplasma-pneumoniae-*Organismen anzeigt.

8. Verfahren zum Nachweis der Anwesenheit oder Abwesenheit von *Mycoplasma-pneumoniae-*Organismen in einer Probe, wobei das Verfahren Folgendes umfasst:
a) Behandeln der Probe unter Verwendung eines Paars von Nucleinsäureprimern in einer Nucleinsäureamplifikationsreaktion, wobei es sich bei dem ersten Primer um ORF6LP2 (SEQ ID Nr. 2) und bei dem zweiten Primer um ORF6RP2 (SEQ ID Nr. 4) handelt; und
b) Nachweisen eines gegebenenfalls amplifizierten Nucleinsäureprodukts, wobei der Nachweis des amplifizierten Produkts die Anwesenheit von *Mycoplasma-pneumoniae-*Organismen anzeigt.

9. Verfahren zum Amplifizieren einer Zielnucleinsäuresequenz eines *Mycoplasma-pneumoniae-*Organismus, umfassend:
a) Hybridisieren von
i) einem ersten Amplifikationsprimer, der aus der Gruppe ausgewählt ist, die aus den Zielbindungssequenzen von ORF6LP1 (SEQ ID Nr. 1), ORF6LP2 (SEQ ID Nr. 2) und ORF6Left PCR (SEQ ID Nr. 12) besteht, und gegebenenfalls einer Sequenz, die für eine Amplifikationsreaktion erforderlich ist; und
ii) einem zweiten Amplifikationsprimer, der aus der Gruppe ausgewählt ist, die aus den Zielbindungssequenzen von ORF6RP1 (SEQ ID Nr. 3), ORF6RP2 (SEQ ID Nr. 4) und ORF6Right PCR (SEQ ID Nr. 13) besteht, und gegebenenfalls einer Sequenz, die für eine Amplifikationsreaktion erforderlich ist;
mit der Nucleinsäure; und
b) Verlängern des hybridisierten ersten und zweiten Amplifikationsprimers an der Zielnucleinsäuresequenz, wodurch die Zielnucleinsäuresequenz amplifiziert wird.

10. Verfahren gemäß Anspruch 7, 8 oder 9, wobei die Nucleinsäureamplifikationsreaktion eine Polymerase-Kettenreaktion (PCR) oder eine Strangverdrängungsamplifikationsreaktion (SDA) ist.

## Revendications

1. Oligonucléotide consistant en une séquence de liaison cible choisie dans le groupe constitué par les séquences de liaison cibles de ORF6LP1 (SEQ ID NO: 1). ORF6LP2 (SEQ ID NO: 2), ORF6Left PCR (SEQ ID NO: 12), ORF6RP1 (SEQ ID NO: 3), ORF6RP2 (SEQ ID NO: 4) et ORF6Right PCR (SEQ ID NO: 13), et optionnellement une séquence nécessaire à une réaction d'amplification.

2. Oligonucléotide de la revendication 1 dans lequel la séquence nécessaire à la réaction d'amplification est un site de reconnaissance par une endonucléase de restriction qui est clivable par une endonucléase de restriction.

3. Oligonucléotide choisi dans le groupe consistant en ORF6ADPT1 (SEO ID NO: 9), un acide nucléique complémentaire de SEQ ID NO: 9, ORF6ADPT2 (SEQ ID NO: 10) et un acide nucléique complémentaire de SEQ ID NO: 10.

4. Paire d'amorces d'amplification comprenant:
a) une première amorce consistant en une séquence de liaison cible choisie dans le groupe constitué par ORF6LP1 (SEC ID NO: 1), ORF6LP2 (SEQ ID NO: 2) et ORF6Left PCR (SEO ID NO: 12), et, optionnellement, une séquence nécessaire à une réaction d'amplification, et;
b) une seconde amorce consistant en une séquence de liaison cible choisie dans le groupe constitué par la séquence de liaison cible de ORF6RP1 (SEO ID NO: 3), ORF6RP2 (SEO ID NO: 4) et ORF6Right PCR (SEQ ID NO: 13), et, optionnellement, une séquence nécessaire à une réaction d'amplification.

5. Kit comprenant
a) une ou plusieurs amorces choisies parmi le groupe constitué par ORF6LP1 (SEQ ID NO: 1), ORF6LP2 (SEQ ID NO: 2) et ORF6Left PCR (SEQ ID NO: 12).
b) une ou plusieurs amorces choisies parmi le groupe constitué par ORF6RP1 (SEQ ID NO: 3), ORF6RP2 (SEQ ID NO: 4) et ORF6Right PCR (SEQ ID NO: 13),
c) une ou plusieurs amorces signal choisies parmi le groupe constitué par ORF6ADPT1 (SEQ ID NO: 9), un acide nucléique complémentaire de SEQ ID NO: 9, ORF6ADPT2 (SEQ ID NO: 10) et un acide nucléique complémentaire de SEQ ID NO: 10.

6. Kit selon la revendication 5 comprenant en outre:
e) une paire d'amorces spécifiques pour l'amplification d'une séquence d'acide nucléique spécifique de *Legionella pneumophila;*
f) une paire d'amorces spécifiques pour l'amplification d'une séquence d'acide nucléique spécifique de *Bordetella pertussis;* et
g) une paire d'amorces spécifiques pour l'amplification d'une séquence d'acide nucléique indicative d'une infection chlamydiale.

7. Procédé de détection de la présence ou de l'absence d'organismes de *Mycoplasma pneumoniae* dans un échantillon, ledit procédé consistant à:
a) traiter ledit échantillon en utilisant une paire d'amorces d'acide nucléique dans une réaction d'amplification d'acides nucléiques où une dite première amorce est ORF6LP1 (SEQ ID NO: 1) et une dite seconde amorce est ORF6RP1 (SEQ ID NO: 3), et
b) détecter tout produit d'acide nucléique amplifié, procédé selon lequel la détection du produit amplifié indique la présence d'organismes *Mycoplasma pneumoniae.*

8. Procédé de détection de la présence ou de l'absence d'organismes de *Mycoplasma pneumoniae* dans un échantillon, ledit procédé consistant à:
a) traiter ledit échantillon en utilisant une paire d'amorces d'acide nucléique dans une réaction d'amplification d'acide nucléique où une dite première amorce est ORF6LP2 (SEQ ID NO: 2) et une dite seconde amorce est ORF6RP2 (SEQ ID NO: 4), et
b) détecter tout produit d'acide nucléique amplifié, procédé selon lequel la détection d'un produit amplifié indique la présence d'organismes *Mycoplasma pneumoniae.*

9. Procédé d'amplification d'une séquence d'acide nucléique cible d'un organisme *Mycoplasma pneumoniae* consistant à:
a) hybrider à l'acide nucléique
i) une première amorce d'amplification choisie dans le groupe constitué par les séquences de liaison cibles de ORF6LP1 (SEQ ID NO: 1), ORF6LP2 (SEQ ID NO: 2) et ORF6Left PCR (SEQ ID NO: 12), et optionnellement, une séquence nécessaire à une réaction d'amplification, et
ii) une seconde amorce d'amplification choisie parmi le groupe constitué par les séquences de liaison cibles de ORF6RP1 (SEQ ID NO: 3), ORF6RP2 (SEQ ID NO: 4) et ORF6Right PCR (SEQ ID NO: 13), et optionnellement, une séquence nécessaire à la réaction d'amplification, et
b) allonger les première et seconde amorces d'amplification de la séquence d'acide nucléique cible de manière à ce que la séquence d'acide nucléique cible soit amplifiée.

10. Procédé selon la revendication 7, 8 ou 9, dans lequel la réaction d'amplification d'acide nucléique est la réaction en chaîne par polymérase (PCR) ou une réaction d'amplification par déplacement de brin (SDA).
